(19) Europäisches Patentamt
European Patent Office
Office européen des brevets

(11) Veröffentlichungsnummer: **0 159 490**
**A1**

(12) **EUROPÄISCHE PATENTANMELDUNG**

(21) Anmeldenummer: **85102236.8**

(22) Anmeldetag: **28.02.85**

(51) Int. Cl.⁴: **D 06 M 16/00**, A 61 L 15/00, A 01 N 25/10, A 01 N 59/20

(30) Priorität: **06.03.84 DE 3408131**

(43) Veröffentlichungstag der Anmeldung: **30.10.85**
**Patentblatt 85/44**

(84) Benannte Vertragsstaaten: **AT BE CH DE FR GB IT LI LU NL SE**

(71) Anmelder: **Chemiefaser Lenzing Aktiengesellschaft, A-4860 Lenzing (AT)**

(72) Erfinder: **Marini, Gerhard, Dr. Ingo, Hauptstrasse 40, A-4860 Lenzing (AT)**
Erfinder: **Sustmann, Scarlet, Dr., Am Nachtigallenwäldchen 34, A-4060 Viersen 12 (AT)**

(54) **Desodorierendes und mikrobistatisches Fasermaterial.**

(57) Ein Fasermaterial aus Cellulosefasern, die durch salzbildende, anionische Gruppen, bevorzugt durch Carboxymethylgruppen, modifiziert sind und die über diese Gruppen an die Faser gebundenes Kupfer enthalten, hat desodorierende und mikrobistatische Eigenschaften. Der Substitutionsgrad der Cellulosefasern sollte wenigstens so hoch sein, dass 0,1 bis 3,0 Gew.-% Kupfer gebunden werden kann. Das Fasermaterial eignet sich für medizinische, hygienische und ästhetische Anwendungen.

EP 0 159 490 A1

Dr.JG/Po

P a t e n t a n m e l d u n g

D 6980 EP

"Desodorierendes und mikrobistatisches Fasermaterial"

Die Erfindung betrifft ein desodorierendes und mikrobistatisches Fasermaterial mit wasseraufsaugenden Eigenschaften. Solche Fasermaterialien sind für zahlreiche medizinische, hygienische und aesthetische Zwecke von großem Wert.

Es ist bekannt, daß in Artikeln, die auf der Körperoberfläche angewendet werden, insbesondere an Stellen verstärkter Schweißabsonderung oder dort, wo Körpersekrete wie Blut oder Urin abgesondert werden, bereits nach kurzer Zeit bakterielle Zersetzung des Sekrets durch ubiquitär vorhandene Bakterien erfolgt und daß dies zu unangenehmer Geruchsbelästigung und in vielen Fällen zu Gefahren durch das Wachstum pathogener Keime führt.

Es hat daher nicht an Versuchen gefehlt, Fasermaterialien mikrobistatisch und desodorierend auszurüsten. Man hat für diesen Zweck bekannte Mikrobizide und Deodorantien wie Jod, Phenole, Thiophenole, quartäre Ammoniumverbindungen, Antibiotika, Nitroverbindungen, Formaldehyd und Schwermetallverbindungen vorgeschlagen. Eines der größten Probleme ist dabei die Fixierung des Wirkstoffs auf der Faser. Man hat auch schon Kupferverbindungen für diesen Zweck vorgeschlagen.

...

Aus DE-OS 31 35 410 ist z.B. ein absorbierender Körper aus Cellulose-Faservlies oder Watte bekannt, der mit der Lösung eines Kupfersalzes besprüht oder aus einer mit einer solchen Lösung behandelten Faser oder Watte angefertigt ist. Dieses Material hat den Nachteil, daß das Kupfersalz nicht gleichmäßig in dem absorbierenden Körper verteilt ist, daß das Kupfersalz nach dem Trocknen teilweise auskristallisiert, was bei der Verarbeitung des Fasermaterials zu Schwierigkeiten führt und daß das Kupfersalz sich in Gegenwart von Wasser, z.B. in einer nassen Windel oder in einem feuchten Tampon löst und so in relativ hoher Konzentration an die Hautoberfläche gelangen und dort zu unerwünschten Reaktionen führen kann.

Aus EP-A 19 371 ist ein blutkoagulierendes Material bekannt, welches aus einem wasserquellbaren, kovalent vernetzten, anionischen Polyelektrolyten, z.B. aus vernetzter Carboxymethylcellulose besteht, der mit Übergangsmetallionen, z.B. mit Kupfer-ionen ausgerüstet ist. Solche Materialien sind aber selbst nicht faserförmig bzw. nicht zu Fasern, Watte oder Vliesstoffen zu verarbeiten, so daß sie bei ihrer Anwendung in Hygienemitteln mit einem Fasermaterial als Träger verarbeitet werden müssen. Sowohl die Herstellung als auch die Verarbeitung dieses Materials ist sehr aufwendig.

Es bestand daher die Aufgabe ein saugfähiges Fasermaterial zu finden, welches dauerhaft, also nicht auswaschbar, desodorierend und mikrobistatisch ausgerüstet ist und auf einfache Weise herstellbar und verarbeitbar ist.

...

Es wurde gefunden, daß diese Aufgabe auf überraschend elegante Weise erfüllt wird durch ein saugfähiges Fasermaterial, wenn das Fasermaterial aus durch anionische, salzbildende Gruppen modifizierten Cellulosefasern besteht und über die anionischen Gruppen an die Faser gebundenes Kupfer enthält. Unter Cellulosefasern werden vor allem Zellstoff, Baumwolle und Viskosefasern verstanden.

Anionische, salzbildende Gruppen lassen sich auf vielerlei Weise in das Cellulosemolekül einbauen. Solche anionisch modifizierte Cellulosefasern sind bekannt, einige davon auch im Handel erhältlich. Geeignet sind z.B. Cellulosefasern, die über den Sauerstoff an die Anhydroglukoseeinheiten gebundene Gruppen der allgemeinen Formel $-PO_3H^{(-)}$, $-(CH_2)_n-PO_3H^{(-)}$, $-(CH_2)_n-SO_3^{(-)}$, $(CH_2)_n-COO^{(-)}$ tragen, wobei n einen Wert von 1 - 3 haben kann. Bekannte Cellulosederivate dieser Art sind z.B. Cellulosephosphat, welches durch Veresterung von Cellulose mit Phosphorsäure zugänglich ist, Phosphonoethylcellulose, die durch Veretherung von Alkalicellulose mit Chlorethylphosphonat erhältlich ist, Phosphonomethylcellulose, die durch Veretherung von Cellulose mit Chlormethylphosphonat hergestellt werden kann, Sulfoethylcellulose, die durch Veretherung von Cellulose mit Chlorethansulfonat zugänglich ist und die analog herstellbare Sulfomethylcellulose und Sulfopropylcellulose. Durch Veretherung mit 1-Chlor-2-hydroxypropansulfonat ist die 1-Sulfo-2-hydroxypropylcellulose erhältlich.

...

Die Einführung von Carboxylgruppen in das Cellulosemolekül ist auf zwei prinzipiell verschiedenen Wegen möglich:

- durch physikalische Inkorporation von Carboxylgruppen tragenden Verbindungen in die Viskose, d.h. in eine als Cellulosexanthogenat gelöste Cellulose, unter Bildung von sogenannter inkorporierter Viskosefasern (Alloy Fibers) oder

- durch chemische Umsetzung (Veretherung) der faserbildenden Cellulose mit Carboxylgruppen tragenden Reagentien unter Bildung von einheitlich mit z.B. Carboxyalkylgruppen der Formel $-(CH_2)_n-COOH$, in der n einen Wert von 1 - 3 haben kann, modifizierten Cellulosefasern.

Die physikalische Inkorporation Carboxylgruppen tragender Verbindungen in die Viskose wird z.B. durch Beimischen von Alkalisalzen von Acrylsäure-Homopolymerisaten, Acrylsäure-Methacrylsäure-Copolymerisaten, Methylvinylether-Maleinsäureanhydrid-Copolymerisaten, Alginsäure oder Carboxymethylcellulose zur Viskoselösung und anschließendes Verspinnen in üblicher Weise in ein Fällungsbad erreicht. Handelsübliche Fasern dieses Typs sind z.B. die ABSORBIT(R) - Fasern von Fa. Enka, die eine Mischfaser aus Viskose und Acrylsäure-Methacrylsäure-Copolymerisat darstellen. Solche

...

Fasern sind nicht einheitlich modifiziert, sondern setzen sich aus modifizierten und unsubstituierten Faserfragmenten zusammen.

Einheitlich chemisch mit Carboxyalkylgruppen modifizierte Cellulosefasern sind für die Herstellung der erfindungsgemäßen Fasermaterialien besonders bevorzugt. In solchen Fasern ist die gesamte faserbildende Cellulose gleichmäßig modifiziert. Sie werden z.B. dadurch erhalten, daß man Cellulosefasern direkt nach Überführung in die Alkalicellulose mit z.B. Natriumchloracetat carboxymethyliert. Die so modifizierte Cellulose kann über das Viskose-Spinnverfahren in ihrer Faserstruktur verbessert werden. Man kann aber auch eine über das Viskose-Spinnverfahren regenerierte Viskosefaser anschliessend mit Chloressigsäure carboxymethylieren. Eine dritte Möglichkeit, zu einheitlich mit Carboxyalkylgruppen modifizierten Cellulosefasern zu gelangen, besteht darin, daß man während der Xanthogenierung der Viskoselösung z.B. Na-Chloracetat zusetzt und dann die carboxymethylierte Viskose wie üblich verspinnt. Solche einheitlich mit Carboxymethylgruppe modifizierte Viskosefasern sind im Handel erhältlich, z.B. unter der Bezeichnung VISCOSORB 1 N von Fa. Chemiefaser Lenzing.

Setzt man der Viskose-Lösung während der Xanthogenierung z.B. Acrylnitril zu, so erhält man nach Ablauf des Viskosespinnprozesses Viskosefasern, die aus niedrig substituierter Carboxyethylcellulose bestehen. Solche Fasern sind unter der Bezeichnung BAR-Fieber (Bondable Avisco Rayon) der FMC-Corporation im Handel.

...

Andere zur einheitlichen Modifizierung der Viskose durch Zusatz zur Viskoselösung während der Xanthogenierung geeignete Reagenzien sind z.B. Natrium-Vinylsulfonat, Na-Chlor-methansulfonat, Na-Chlormethanphosphonat. Man erhält auf diese Weise einheitlich chemisch modifizierte Viskosefasern mit Sulfoethylgruppen, Sulfomethylgruppen und Phosphonomethylgruppen.

Zur Herstellung des erfindungsgemäßen Fasermaterials ist jedoch eine mit Carboxylgruppen, insbesondere mit Carboxymethylgruppen einheitlich chemisch modifizierte Cellulosefaser besonders bevorzugt, insbesondere ein Fasermaterial, welches aus einer durch das Viskose-Spinnverfahren regenerierten, mit Carboxymethylgruppen modifizierten Cellulose erhalten wird.

Der Substitutionsgrad der für das erfindungsgemäße Fasermaterial geeigneten Cellulosederivate sollte in bezug auf die anionischen, salzbildenden Gruppen so hoch sein, daß sie 0,1 bis 3,0 Gew.-% Kupfer, bezogen auf das Gewicht des Fasermaterials, binden können. Die für das erfindungsgemäße Fasermaterial am besten geeigneten, mit Carboxymethylgruppen modifizierten Viskosefasern weisen einen Substitutionsgrad von 0,01 bis 0,3 auf, d.h. sie enthalten im Mittel etwa 0,01 bis 0,3 Carboxymethylgruppen pro Anhydroglukoseeinheit. Der Gehalt an gebundenem Kupfer sollte von 0,2 - 2,0 Gew.-%, bevorzugt von 0,6 - 1,6 Gew.-% des Fasermaterials ausmachen.

...

Es hat sich weiterhin als vorteilhaft erwiesen, wenn das erfindungsgemäße Fasermaterial einen Faser-pH-Wert, gemessen nach DIN 54275, von 4 - 5 aufweist. Ein solcher Faser-pH-Wert bewirkt, daß die erfindungsgemäßen Fasermaterialien einen gewissen Puffereffekt gegenüber aufgesaugten Körperflüssigkeiten haben und so den physiologisch günstigen, schwach sauren pH-Wert der Hautoberfläche herstellen, wodurch Reizungen und Anfälligkeit gegenüber Erkrankungen vermieden werden.

Da das erfindungsgemäße Fasermaterial in erster Linie zur Herstellung von sanitären Hygienemitteln geeignet ist, sollte es in einer weiteren bevorzugten Ausführungsform ein hohes Wasserrückhaltevermögen, gemessen nach DIN 53814, von mindestens 80 % aufweisen.

Die Herstellung des erfindungsgemäßen Fasermaterials ist aus bekannten Fasern mit anionischen, salzbildende Gruppen enthaltenden Fasern in einfacher Weise dadurch möglich, daß man

- die anionische, salzbildende Gruppen enthaltenden Fasern mit einer wäßrigen Kupfer-II-salzlösung behandelt und

- die Fasern mit Wasser weitgehend salzfrei wäscht und trocknet.

Das bevorzugte Fasermaterial mit hohem Wasserückhaltevermögen läßt sich sehr leicht dadurch herstellen, daß man eine Carboxymethyl-Viskosefaser mit einem Substitutionsgrad von 0,01 - 0,3 und mit entsprechend

...

hohen Wasserrückhaltevermögen, z.B. die handelsüblichen Fasern Viscosorb(R) 1S mit einem Wasserrückhaltevermögen von ca. 200 % und einem Substitutionsgrad von ca. 0,10 entweder in der Natriumsalzform oder nach Überführung in die freie Säureform in das beschriebene Verfahren einsetzt. Als wäßrige Kupfer-II-salzlösung eignet sich z.B. eine Lösung von 1 - 20 g/l $CuSO_4 \cdot 5H_2O$ in Wasser. Die Behandlung erfolgt im allgemeinen ohne Wärmezufuhr über einen Zeitraum von wenigstens 1 Minute und ist in höchstens 1 Stunde beendet. Danach wird die Kupfersalzlösung , z.B. durch Abpressen, von der Faser abgetrennt, die Faser mit Wasser solange gewaschen, bis das Waschwasser weitgehend frei von Sulfationen ist, das Wasser, z.B. durch Abpressen, von der Faser abgetrennt und die Faser im Luftstrom getrocknet. Die Erzeugung eines erfindungsgemäßen Fasermaterials mit einem Faser-pH-Wert von 4 - 5 läßt sich nach diesem Verfahren leicht dadurch erreichen, daß man eine auf einen pH-Wert von 4 - 5 eingestellte Kupfer-II-salzlösung verwendet.

Das erfindungsgemäße Fasermaterial weist eine von der Menge des gebundenen Kupfers abhängige, mehr oder weniger ausgeprägte Blaufärbung auf, die unter Anwendungsbedingungen nicht auswaschbar ist. Die Farbe ist im allgemeinen nicht störend und entspricht hygienischen Vorstellungen. Bakterien, insbesondere solche, die im Intimbereich auftreten, z.B. Escherichia coli, Staphylococcus aureus und Candida albicans zeigen auf dem erfindungsgemäßen Fasermaterial selbst unter optimalen Brutbedingungen keine Vermehrung. Auch wiesen die mit den erfindungsgemäßen Fasermaterialien beschickten Nährkulturen selbst nach dreitägiger Inkubation

...

nicht den unangenehmen, charakteristischen Geruch auf, den entsprechende Kulturen, die mit Normalwatte beschickt waren, aufwiesen. Aufgrund der genannten Eigenschaften eignet sich das erfindungsgemäße Fasermaterial für zahlreiche Anwendungen, insbesondere für medizinische, hygienische und aesthetische Zwecke, z.B. für Wundauflagen, Schweißpolster, Windeln und andere , saugfähige Einwegartikel.

Die folgenden Beispiele sollen den Gegenstand der Erfindung näher erläutern:

...

# Beispiele

A. Herstellung der erfindungsgemäßen, desodorierenden und mikrobistatischen Fasermaterialien

Die Beispiele 1 - 4 wurden ausgehend von einer handelsüblichen, carboxymethylierten Viskosefaser - Viscosorb 1 S(R) der Firma Chemiefaser Lenzing AG - mit folgenden technischen Daten hergestellt.

Viscosorb 1 S(R)

| | |
|---|---|
| Faser-pH-Wert: | 7,0 - 7,5 (DIN 54275) |
| Carboxylgruppengehalt: | 2,3 - 2,7 Gew.-% |
| Substitutionsgrad: | 0,09 - 0,10 |
| Wasserrückhaltevermögen: | 180 - 200 % |

Beispiel 1

1 kg Fasern der Type Viscosorb 1 S wurde mit 20 l einer Lösung von 20 g $CuSO_4 \cdot 5H_2O$ in 1000 ml Wasser, der pH-Wert mit verdünnter Schwefelsäure auf 5 eingestellt war, 30 Minuten lang bei Raumtemperatur (20°C) behandelt. Anschließend wurde das Fasermaterial auf eine Feuchte von 200 % abgepreßt und solange mit Wasser gewaschen, bis das Waschwasser sulfatfrei war. Danach wurde wiederum auf eine Feuchte von ca. 200 % abgepreßt und 4 Stunden im Umlufttrockenschrank bei 105°C getrocknet. Das erhaltene Fasermaterial hatte folgende Daten:

| | | |
|---|---|---|
| Faser-pH-Wert | : | 5,6 (DIN 54275) |
| Kupfer-Gehalt | : | 1,45 Gew.-% |

...

Beispiel 2

Es wurde wie in Beispiel 1 verfahren, mit dem Unterschied, daß der pH-Wert der Kupfersulfat-Lösung auf 4 eingestellt wurde.

Das erhaltene Fasermaterial hatte folgende Daten:

| | | |
|---|---|---|
| Faser-pH-Wert | : | 5,0 (DIN 54275) |
| Kupfer-Gehalt | : | 2,92 Gew.-% |

Beispiel 3

1 kg Fasern (Viscosorb 1S$^{(R)}$) wurde zunächst durch Behandlung mit einer 0,2 %igen wäßrigen Salzsäurelösung (30 Minuten bei Raumtemperatur) und Auswaschen der überschüssigen Salzsäure in die Säureform überführt. Die auf eine Feuchte von ca. 200 % abgepreßten Fasern wurden dann mit einer Lösung von 20 g $CuSO_4 \cdot 5H_2O$ in 1000 ml Wasser, deren pH-Wert auf 4,5 mit verdünnter Schwefelsäure eingestellt war, 30 Minuten lang bei Raumtemperatur (20$^{o}$ C) behandelt.

Die Aufarbeitung erfolgte analog Beispiel 1 und 2. Das erhaltene Fasermaterial hatte folgende Daten:

| | | |
|---|---|---|
| Faser-pH-Wert | : | 4,5 (DIN 54275) |
| Kupfer-Gehalt | : | 1,65 Gew.-% |
| Wasserrückhaltevermögen: | | 87,4 % (DIN 53 814) |

Beispiel 4

Es wurde wie in Beispiel 3 verfahren, mit dem Unterschied, daß die Behandlung mit einer Lösung von 5,0 g $CuSO_4 \cdot 5H_2O$ in 1000 ml Wasser erfolgte.

...

Das erhaltene Fasermaterial hatte folgende Daten:

| | | |
|---|---|---|
| Faser-pH-Wert | : | 4,2 (DIN 54 275) |
| Kupfer-Gehalt | : | 0,64 |
| Wasserrückhaltevermögen: | | 1o9,5 (DIN 53814) |

Beispiel 5 (Vergleichsbeispiel)

1 kg Normalviskosefasern wurden analog Beispiel 1 behandelt. Das erhaltene Fasermaterial hatte folgende Daten:

| | | |
|---|---|---|
| Faser-pH-Wert | : | 6,4 (DIN 54 275) |
| Kupfer-Gehalt | : | 0,05 Gew.-% |

B. Nachweis der Unterbindung bakteriellen Wachstums und der desodorierenden Wirkung

Geprüfte Keime: Staph. aureus
E. coli
Ps. aeruginosa
Proteus mirabilis
Candida albicans

Versuchsdurchführung:

Je 1 g Normalviskose (3.6 dtex, 30 mm) der Fa. Hoechst und mit Kupfer ausgerüstetes Fasermaterial gemäß Beispiel 3 und 4 wurden mit jeweils 10 ml CASO-Bouillon in Reagenzgläsern versetzt. Die so präparierten Reagenzgläser wurden mit jeweils 0,1 ml Keimsuspension (s.o.) - 24 h Bouillonkultur 1 : 100 verdünnt - geimpft und bei 37°C inkubiert.

...

Ergebnis:

Im Falle der Normalviskose trat mit jedem Prüfstamm (s.o.) Wachstum ein, während bei den mit Kupfer ausgerüsteten Fasermaterialien auch nach 72 h in keinem Fall Wachstum festgestellt werden konnte. Gleichzeitig wurde auch nur im Falle der mit Normalviskose beschickten Ansätze eine Geruchsentwicklung beobachtet, die der normaler Bouillonkulturen der entsprechenden Keime entsprach. Bei den Ansätzen mit den Spezialwatten fehlte diese Geruchsbildung.

...

## Patentansprüche

1. Desodorierendes und mikrobistatisches, saugfähiges Fasermaterial, dadurch gekennzeichnet, daß das Fasermaterial aus durch anionische, salzbildende Gruppen modifizierten Cellulosefasern besteht und über die anionischen Gruppen an die Faser gebundenes Kupfer enthält.

2. Fasermaterial nach Anspruch 1, dadurch gekennzeichnet, daß die Cellulosefasern über den Sauerstoff an die Anhydroglucoseeinheiten gebundene Gruppen der allgemeinen Formel

$$-PO_3H^{(-)},\ -(CH_2)_n-PO_3H^{(-)},\ -(CH_2)_n-SO_3^{(-)},\ \text{oder}$$

$$-(CH_2)_n - COO^{(-)}$$

tragen, in welchen n einen Wert von 1 - 3 hat, wobei der Substitutionsgrad der Cellulose wenigstens so hoch ist, daß sie 0,1 bis 3,0 Gew.-% Kupfer, bezogen auf das Gewicht des Fasermaterials binden kann.

3. Fasermaterial nach Anspruch 1 oder 2, dadurch gekennzeichnet, daß das Fasermaterial aus einer einheitlichen, mit Carboxymethylgruppen modifizierten Viskosefaser besteht.

4. Fasermaterial nach Anspruch 3, dadurch gekennzeichnet, daß die modifizierte Viskosefaser einen Substititionsgrad von 0,01 bis 0,3 aufweist und der Gehalt an gebundenem Kupfer von 0,2 - 2,0 Gew.-%, bevorzugt von 0,6 - 1,6 Gew.-% des Fasermaterials ausmacht.

...

5. Fasermaterial nach Anspruch 1 - 4, dadurch gekennzeichnet, daß es einen Faser-pH-Wert von 4 - 5 aufweist.

6. Fasermaterial nach Anspruch 1 - 5, dadurch gekennzeichnet, daß es ein Wasserrückhaltevermögen von mehr als 80 % aufweist.

7. Verfahren zur Herstellung von desodorierendem und mikrobistatischem Fasermaterial, dadurch gekennzeichnet, daß man

- anionische salzbildende Gruppen enthaltende Fasern mit einer wäßrigen Kupfer-II-salzlösung behandelt,

- die Fasern mit Wasser weitgehend salzfrei wäscht und trocknet.

8. Verfahren nach Anspruch 7, dadurch gekennzeichnet, daß man

- eine Carboxymethyl-Viskosefaser mit einem Substitutionsgrad von 0,01 - 0,3 oder deren Alkalisalz mit einer Lösung von 1 - 20 g/l $CuSO_4 \cdot 5H_2O$ in Wasser ohne Wärmezufuhr für wenigstens 1 Minute behandelt,

  - die Kupfer-II-salzlösung von der Faser abtrennt,
  - die Faser mit Wasser so lange wäscht, bis das Waschwasser weitgehend frei von Sulfationen ist,
  - das Wasser von der Faser abtrennt die die Fasern im Luftstrom trocknet.

...

9. Verfahren nach Anspruch 7 oder 8, dadurch gekennzeichnet, daß man die Fasern mit einer, auf einen pH-Wert von 4 - 5 eingestellten, wäßrigen Kupfer-II-salzlösung behandelt.

Europäisches Patentamt

## EUROPÄISCHER RECHERCHENBERICHT

0159490 Nummer der Anmeldung

EP 85 10 2236

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-1 504 077 (THE CALICO PRINTERS ASSOC.) * Zusammenfassung * | 1-9 | D 06 M 16/00<br>A 61 L 15/00<br>A 01 N 25/10<br>A 01 N 59/20 |
| X | FR-A-1 499 358 (MOSKOVSKY TEKSTILNY INSTITUT) * Beispiel 2 * | 1 | |
| X | FR-A-1 499 788 (MOSKOVSKY TEXTILNY INSTITUT) * Beispiel 3 * | 1,2 | |
| A | US-A-2 856 330 (H.N.VAGENIUS) * Ansprüche 1-4 * | 1 | |
| D,A | GB-A-2 083 748 (LANDSTINGENS INKÖPSCENTRAL) * Ansprüche 1,6 * | 1 | **RECHERCHIERTE SACHGEBIETE (Int. Cl.4)**<br>D 06 M<br>A 61 L<br>A 01 N |

Der vorliegende Recherchenbericht wurde für alle Patentansprüche erstellt.

| Recherchenort | Abschlußdatum der Recherche | Prüfer |
|---|---|---|
| DEN HAAG | 25-06-1985 | PELTRE CHR. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82